(19) Europäisches Patentamt
European Patent Office
Office européen des brevets

(11) **EP 2 451 769 B1**

(12) **EUROPÄISCHE PATENTSCHRIFT**

(45) Veröffentlichungstag und Bekanntmachung des Hinweises auf die Patenterteilung:
**07.01.2015 Patentblatt 2015/02**

(21) Anmeldenummer: **10729890.3**

(22) Anmeldetag: **08.07.2010**

(51) Int Cl.:
*C25B 3/00* *(2006.01)* *C07C 209/02* *(2006.01)*

(86) Internationale Anmeldenummer:
**PCT/EP2010/059771**

(87) Internationale Veröffentlichungsnummer:
**WO 2011/003964 (13.01.2011 Gazette 2011/02)**

(54) **VERFAHREN ZUR DIREKTAMINIERUNG VON KOHLENWASSERSTOFFEN ZU AMINOKOHLENWASSERSTOFFEN MIT ELEKTROCHEMISCHER ABTRENNUNG VON WASSERSTOFF**

PROCESS FOR DIRECT AMINATION OF HYDROCARBONS TO AMINO HYDROCARBONS WITH ELECTROCHEMICAL SEPARATION OF HYDROGEN

PROCÉDÉ D'AMINATION DIRECTE D'HYDROCARBURES EN AMINO-HYDROCARBURES AVEC SÉPARATION ÉLECTROCHIMIQUE D'HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **10.07.2009 EP 09165219**

(43) Veröffentlichungstag der Anmeldung:
**16.05.2012 Patentblatt 2012/20**

(73) Patentinhaber: **BASF SE**
**67056 Ludwigshafen (DE)**

(72) Erfinder:
• **KUBANEK, Petr**
**68163 Mannheim (DE)**
• **PANCHENKO, Alexander**
**67063 Ludwigshafen (DE)**
• **FISCHER, Andreas**
**64646 Heppenheim (DE)**
• **HEIDEMANN, Thomas**
**68519 Viernheim (DE)**

(56) Entgegenhaltungen:
**WO-A1-00/09473 WO-A1-2007/025882**

• **DARLING A S ET AL: "Trennung und Reinigung von Wasserstoff durch Permeation an Membranen aus Palladium-Legierungen", CHEMIE INGENIEUR TECHNIK, WILEY VCH. VERLAG, WEINHEIM; DE, Bd. 1, Nr. 37, 1. Januar 1965 (1965-01-01), Seiten 18-27, XP002413380, ISSN: 0009-286X, DOI: DOI:10.1002/CITE. 330370105**

Bemerkungen:
Die Akte enthält technische Angaben, die nach dem Eingang der Anmeldung eingereicht wurden und die nicht in dieser Patentschrift enthalten sind.

EP 2 451 769 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Direktaminierung von Kohlenwasserstoffen mit einem Aminierungsreagenz zu Aminokohlenwasserstoffen in Gegenwart eines Katalysators, wobei mindestens ein Teil des bei der Umsetzung entstehenden Wasserstoffs mittels einer gasdichten Membran-Elektroden-Assembly elektrochemisch abgetrennt wird.

Die kommerzielle Herstellung von Aminokohlenwasserstoffen aus Kohlenwasserstoffen wird üblicherweise in mehrstufigen Reaktionen durchgeführt. So wird bei der Herstellung von Anilin aus Benzol zunächst ein Benzolderivat wie Nitrobenzol, Chlorbenzol oder Phenol hergestellt, welches anschließend in ein- oder mehrstufigen Reaktionen zu Anilin umgesetzt wird.

Es sind auch Verfahren zur direkten Herstellung von Aminokohlenwasserstoffen aus den entsprechenden Kohlenwasserstoffen bekannt. Reaktionen, bei denen Aminokohlenwasserstoffe direkt aus den entsprechenden Kohlenwasserstoffen hergestellt werden bezeichnet man als Direktaminierung. Wibaut beschreibt 1917 erstmals die heterogenkatalysierte Direktaminierung von Benzol (Berichte 1917, 50, 541-546).

Bei der Direktaminierung findet die Umsetzung des eingesetzten Kohlenwasserstoffs zum entsprechenden Aminokohlenwasserstoff unter Freisetzung von Wasserstoff statt. Bei der Direktaminierung von Benzol zu Anilin entstehen aus 1 mol Benzol und 1 mol Ammoniak 1 mol Anilin und 1 mol Wasserstoff. Die Direktaminierung von Benzol zu Anilin ist durch die Lage des thermodynamischen Gleichgewichts limitiert. Der Gleichgewichtsumsatz liegt bei Temperaturen von 350 °C bei ca. 0,5 Mol% bezogen auf Benzol.

Aufgrund des niedrigen Gleichgewichtsumsatzes ist zur wirtschaftlichen Durchführung der Direktaminierung eine Verschiebung der Lage des thermodynamischen Gleichgewichts auf die Seite der Aminokohlenwasserstoffe notwendig. Diese Verschiebung kann durch eine Abtrennung von Wasserstoff aus dem Reaktionsgemisch der Direktaminierung erreicht werden.

Eine Möglichkeit besteht darin, den bei der Direktaminierung entstehenden Wasserstoff direkt aus der Reaktionszone zu entfernen.

Eine weitere Möglichkeit besteht darin, aus dem Reaktionsgemisch der Direktaminierung, welches Aminokohlenwasserstoff, nicht umgesetzte Edukte und Wasserstoff enthält, einen Teil des Wasserstoffs abzutrennen und die nicht umgesetzten Edukte erneut in die Direktaminierungsreaktion einzusetzten. Die Abtrennung des Wasserstoffs aus dem Reaktionsgemisch ist notwendig, da sonst durch den Wasserstoff das thermodynamische Gleichgewicht in Richtung der Edukte verschoben wird, wodurch die Ausbeute an Aminokohlenwasserstoff bei der erneuten Direktaminierung noch niedriger ausfällt.

Zur Entfernung des Wasserstoffs aus dem Reaktionsgemisch sind mehrere Verfahren beschrieben.

WO 2007/096297 und WO 2000/69804 beschreiben ein Verfahren zur Direktaminierung von aromatischen Kohlenwasserstoffen zu den entsprechenden Aminokohlenwasserstoffen, wobei der entstehende Wasserstoff durch Oxidation an reduzierbaren Metalloxiden aus dem Reaktionsgemisch entfernt wird. Diese Verfahren haben den Nachteil, dass die reduzierbaren Metalloxide nach einiger Zeit wieder mit Sauerstoff regeneriert werden müssen. Dies bedeutet kostenintensive Unterbrechungen des Verfahrens, da die Direktaminierung der Kohlenwasserstoffe und die Regenerierung der reduzierbaren Metalloxide üblicherweise nicht bei den gleichen Bedingungen ablaufen. Zur Regenerierung des Katalysators muss der Reaktor daher üblicherweise entspannt, gespült und inertisiert werden.

Eine weiter unerwünschte Nebenreaktion, die bei der Direktaminierung von Kohlenwasserstoffen zu Aminokohlenwasserstoffen auftritt, ist die Zersetzung von Ammoniak zu Wasserstoff. Diese Zersetzung ist nachteilig, da einerseits das Edukt Ammoniak verloren geht und andererseits der bei der Zersetzung entstehende Wasserstoff zu einer weiteren ungünstigen Verschiebung der Gleichgewichtslage in Richtung der Edukte führt. Bei den in WO 2007/096297 und WO 2000/69804 beschriebenen Katalysatoren nimmt die unerwünschte Zersetzung von Ammoniak mit steigendem Reduktionsgrad der Metalloxide zu, so dass mit steigendem Reduktionsgrad die Gleichgewichtslage immer weiter in Richtung der Edukte verschoben wird.

WO 2007/099028 beschreibt ein Direktaminierungsverfahren von aromatischen Kohlenwasserstoffen zu den entsprechenden Aminokohlenwasserstoffen, wobei in einem ersten Schritt die heterogenkatalysierte Direktaminierung durchgeführt wird und in einem zweiten Schritt der im ersten Schritt entstandene Wasserstoff durch die Umsetzung mit einem Oxidationsmittel wie Luft, Sauerstoff, CO, $CO_2$, NO und/oder $N_2O$ umgesetzt wird. Die Verwendung von Oxidationsmitteln wie Sauerstoff führt zur Oxidation von Ammoniak und zur Bildung weiterer Nebenprodukte. Dies führt zu höheren Stoffkosten und zu zusätzlichen Aufarbeitungsschritten, wodurch die Wirtschaftlichkeit des Verfahrens verschlechtert wird.

WO 2008/009668 beschreibt ebenfalls ein Verfahren zur Direktaminierung von aromatischen Kohlenwasserstoffen. Die Entfernung des Wasserstoffs aus dem Reaktionsgemisch wird hier dadurch erreicht, dass die Direktaminierung unter Zusatz von Verbindungen durchgeführt wird, die mit dem bei der Direktaminierung entstehenden Wasserstoff reagieren. Als der Direktaminierung zugesetzte Verbindungen werden beispielsweise Nitrobenzol und Kohlenmonoxid beschrieben. Auch bei diesem Verfahren treten die vorstehend beschriebenen Nachteile auf.

WO 2007/025882 beschreibt die Direktaminierung von aromatischen Kohlenwasserstoffen zu den entsprechenden Aminokohlenwasserstoffen, wobei Wasserstoff aus dem Reaktionsgemisch physikalisch abgetrennt wird. Die Abtrennung erfolgt hier durch eine selektiv wasserstoffdurchlässige Membran, das heißt Wasserstoff wandert als $H_2$-Molekül durch die Membran. Als Membranmaterialien werden bevorzugt Palladium und Palladiumlegierungen eingesetzt. Die Diffusionsgeschwindigkeit hängt bei diesem Verfahren vom Partialdruckunterschied des Wasserstoffs zwischen der Retentat- und Permeat-Seite der Membran ab. Um höhere Diffusionsgeschwindigkeiten zu erreichen, muss bei höheren Druckunterschieden gearbeitet werden, die hohe Anforderungen an die mechanische Stabilität der Membran stellen. In Basile, Top. Catal. (2008), 51, 107-122, wird zudem beschrieben, dass zum Erreichen einer ausreichend hohen Diffusionsgeschwindigkeit Temperaturen oberhalb 300 °C erforderlich sind. Darüber hinaus müssen zur Ausbildung der Druckunterschiede entsprechende Vorrichtungen zum Verdichten und Expandieren des Gasgemisches vorhanden sein. Aus thermodynamischen Gründen bleibt zudem stets ein gewisser Anteil des Wasserstoffs in dem Retentat zurück. Dies wirkt sich negativ auf die Lage des thermodynamischen Gleichgewichts aus.

Aufgabe der vorliegenden Erfindung ist es daher, ein Verfahren zur Direktaminierung von Kohlenwasserstoffen zu Aminokohlenwasserstoffen bereitzustellen, bei dem Wasserstoff aus dem Reaktionsgemisch möglichst effektiv abgetrennt wird, und dass die vorstehend genannten Nachteile aus dem Stand der Technik bekannten Verfahren zur Direktaminierung vermeidet. Darüber hinaus soll das Verfahren eine Verschiebung der Lage des thermodynamischen Gleichgewichts auf die Seite der Aminokohlenwasserstoffe ermöglichen. Die eingesetzten Kohlenwasserstoffe sollen ebenso wie die bei der Umsetzung anfallenden Nebenprodukte effizient genutzt werden. Das Verfahren soll eine möglichst günstige Energiebilanz und einen möglichst geringen apparativen Aufwand aufweisen.

Die Aufgabe wird erfindungsgemäß gelöst durch ein Verfahren zur Direktaminierung von Kohlenwasserstoffen zu Aminokohlenwasserstoffen umfassend die Schritte:

a) Umsetzen eines Eduktstroms E, der mindestens einen Kohlenwasserstoff und mindestens ein Aminierungsreagenz enthält, zu einem Reaktionsgemisch R enthaltend Aminokohlenwasserstoffe und Wasserstoff, und

b) elektrochemische Abtrennung mindestens eines Teils des bei der Umsetzung entstandenen Wasserstoffs aus dem Reaktionsgemisch R mittels einer gasdichten Membran-Elektroden-Assembly, die mindestens eine selektiv protonleitende Membran und auf jeder Seite der Membran mindestens einen Elektrodenkatalysator aufweist, wobei auf der Retentatseite der Membran mindestens ein Teil des Wasserstoffs an dem Anodenkatalysator zu Protonen oxidiert wird und die Protonen nach Durchqueren der Membran auf der Permeatseite an dem Kathodenkatalysator gemäß

b1) zu Wasserstoff reduziert werden und/oder
b2) mit Sauerstoff zu Wasser umgesetzt werden, wobei der Sauerstoff aus einem Sauerstoff enthaltenden Strom O stammt, der mit der Permeatseite der Membran in Kontakt gebracht wird.

Gegenüber den bekannten Verfahren, bei denen der Wasserstoff durch reduzierbare Metalloxide entfernt wird, weist das erfindungsgemäße Verfahren den Vorteil auf, dass aufwändige und kostenintensive Unterbrechungen des Verfahrens zur Direktaminierung vermieden werden und dass das Verfahren über einen längeren Zeitraum kontinuierlich betrieben werden kann. Das erfindungsgemäße Verfahren kommt zudem im Vergleich zu bekannten Verfahren bei denen gasförmige Oxidationsmittel wie Luft, Sauerstoff, CO, $CO_2$, NO oder $N_2O$ oder Verbindungen wie Nitrobenzol eingesetzt werden ohne eine apparativ aufwändige und kostenintensive Abtrennung der durch die Zugabe der Oxidationsmittel entstandenen Nebenprodukte aus.

Das erfindungsgemäße Verfahren hat gegenüber den im Stand der Technik beschriebenen Verfahren den Vorteil, dass der Wasserstoff elektrochemisch aus dem Wasserstoff erhaltenden Reaktionsgemisch R abgetrennt wird. Die Triebkraft der Wasserstoffabtrennung beruht entweder auf einem Potentialunterschied zwischen den beiden Seiten der selektiv für Protonen durchlässigen Membran (Alternative b1)), oder auf der negativen freien Enthalpie der Reaktion von Wasserstoff und Sauerstoff zu Wasser (Alternative b2)).

Durch den Einsatz selektiv protonenleitender Membranen kann das Verfahren weitgehend unabhängig von Druckunterschieden, wie sie bei der Verwendung von selektiv Wasserstoffmolekül durchlässigen Membranen notwendig sind, betrieben werden. Dadurch kann die Wasserstoffabtrennung bei niedrigeren Drücken und Druckunterschieden durchgeführt werden, wobei vorzugsweise auf einen von außen aufgeprägten Druckunterschied völlig verzichtet wird. Dadurch wird die mechanische Beanspruchung der Membran deutlich verringert, was zu einer Erhöhung ihrer Langzeitstabilität führt. Darüber hinaus vergrößert sich die Auswahl an für die Membran in Frage kommenden Materialien.

Die elektrochemische Abtrennung von Wasserstoff ist im Vergleich zu der Abtrennung von Wasserstoff mittels einer wasserstoffselektiven Membran deutlich effektiver. Daher kann die erforderliche Membranfläche verkleinert werden oder bei gleicher Membranfläche deutlich mehr Wasserstoff aus dem Reaktionsgemisch abgetrennt werden. Die im Reaktionsgemisch nach Abtrennung zurückbleibende Menge an Wasserstoff ist dabei deutlich geringer als bei der Abtrennung mittels einer wasserstoffselektiven Membran.

Wird das erfindungsgemäße Verfahren gemäß Alternative b1) betrieben, wird bei dem Verfahren sehr reiner Wasserstoff gewonnen. Sehr reiner Wasserstoff kann in vielen weiteren Reaktionen oder Prozessen, die auf Verunreinigungen sensibel reagieren eingesetzt werden und stellt somit ein wertvolles Nebenprodukt dar.

Wird das erfindungsgemäße Verfahren gemäß Alternative b2) betrieben, wird bei dem Verfahren elektrische Energie und Wärme frei. Diese Energie kann zum Betrieb des erfindungsgemäßen Verfahrens genutzt werden. Dadurch wird die Energiebilanz des erfindungsgemäßen Verfahrens verbessert.

Je nach Betriebsweise kann so vom Anwender gesteuert werden, ob mehr oder ausschließlich Wasserstoff oder mehr oder ausschließlich elektrische Energie und Wärme bei dem Verfahren gewonnen wird, insbesondere kann die zur Abtrennung des Wasserstoffs gemäß Alternative b1) erforderliche elektrische Energie durch die gleichzeitige Abtrennung von Wasserstoff gemäß Alternative b2) bereit gestellt werden.

Im Folgenden wird die Erfindung ausführlich beschrieben.

Kohlenwasserstoffe:

Erfindungsgemäß enthält der Eduktstrom E mindestens einen Kohlenwasserstoff. Geeignete Kohlenwasserstoffe, die in das erfindungsgemäße Verfahren eingesetzt werden können, sind beispielsweise Kohlenwasserstoffe, wie aromatische Kohlenwasserstoffe, aliphatische Kohlenwasserstoffe und cycloaliphatische Kohlenwasserstoffe, die beliebig substituiert sein können und innerhalb ihrer Kette bzw. ihres Rings/ihrer Ringe Heteroatome und Doppel- oder Dreifachbindungen aufweisen können. Bevorzugt werden in dem erfindungsgemäßen Aminierungsverfahren aromatische Kohlenwasserstoffe und heteroaromatische Kohlenwasserstoffe eingesetzt.

Geeignete aromatische Kohlenwasserstoffe sind beispielsweise ungesättigte cyclische Kohlenwasserstoffe, die einen oder mehrere Ringe aufweisen und ausschließlich aromatische C-H-Bindungen enthalten. Bevorzugte aromatische Kohlenwasserstoffe weisen einen oder mehrere 5- und/oder 6-gliedrige Ringe auf.

Unter einem heteroaromatischen Kohlenwasserstoff sind solche aromatischen Kohlenwasserstoffe zu verstehen, in denen einer oder mehrere der Kohlenstoffatome des aromatischen Rings durch ein Heteroatom ausgewählt aus N, O und S ersetzt ist/sind.

Die aromatischen Kohlenwasserstoffe bzw. die heteroaromatischen Kohlenwasserstoffe können substituiert oder unsubstituiert sein. Unter einem substituierten aromatischen bzw. heteroaromatischen Kohlenwasserstoff sind Verbindungen zu verstehen, in denen ein oder mehrere Wasserstoffatome, die an ein Kohlenstoff- und/oder Heteroatom des aromatischen Rings gebunden sind/ist, durch einen anderen Rest ausgetauscht ist/sind. Geeignete Reste sind beispielsweise substituierte oder unsubstituierte Alkyl-, Alkenyl-, Alkinyl-, Heteroalkyl-, Heteroalkenyl-, Heteroalkinyl-, Cycloalkyl- und/oder Cycloalkinylreste; Halogen, Hydroxy, Alkoxy, Aryloxy, Amino, Amido, Thio und Phosphino. Bevorzugte Reste der aromatischen bzw. heteroaromatischen Kohlenwasserstoffe sind ausgewählt aus $C_{1-6}$-Alkyl, $C_{1-6}$-Alkenyl, $C_{1-6}$-Alkinyl, $C_{3-8}$-Cycloalkyl, $C_{3-8}$-Cycloalkenyl, Alkoxy, Aryloxy, Amino und Amido, wobei sich die Angabe $C_{1-6}$ auf die Anzahl der Kohlenstoffatome in der Hauptkette des Alkylrests, des Alkenylrests oder des Alkinylrests bezieht und die Bezeichnung $C_{3-8}$ auf die Anzahl der Kohlenstoffatome des Cycloalkyl- bzw. Cycloalkenylrings. Es ist weiterhin möglich, dass die Substituenten (Reste) des substituierten aromatischen oder heteroaromatischen Kohlenwasserstoffs ihrerseits weitere Substituenten aufweisen.

Die Zahl der Substituenten (Reste) des aromatischen bzw. heteroaromatischen Kohlenwasserstoffs ist beliebig. In einer bevorzugten Ausführungsform weist der aromatische bzw. heteroaromatische Kohlenwasserstoff jedoch mindestens ein Wasserstoffatom auf, das direkt an ein Kohlenstoffatom oder ein Heteroatom des aromatischen oder heteroaromatischen Rings gebunden ist. Somit weist ein 6-gliedriger Ring bevorzugt 5 oder weniger Substituenten (Reste) auf und ein 5-gliedriger Ring bevorzugt 4 oder weniger Substituenten (Reste). Besonders bevorzugt trägt ein 6-gliedriger aromatischer bzw. heteroaromatischer Ring 4 oder weniger Substituenten, ganz besonders bevorzugt 3 oder weniger Substituenten (Reste). Ein 5-gliedriger aromatischer oder heteroaromatischer Ring trägt bevorzugt 3 oder weniger Substituenten (Reste), besonders bevorzugt 2 oder weniger Substituenten (Reste).

In einer besonders bevorzugten Ausführungsform des erfindungsgemäßen Verfahrens wird ein aromatischer bzw. heteroaromatischer Kohlenwasserstoff der allgemeinen Formel

$$(A)-(B)_n$$

eingesetzt, worin die Symbole die folgende Bedeutung haben:

A    unabhängig Aryl oder Heteroaryl, bevorzugt ist A ausgewählt aus Phenyl, Diphenyl, Benzyl, Dibenzyl, Naphthyl, Anthracen, Pyridyl und Chinolin;

n    eine Zahl von 0 bis 5, bevorzugt 0 bis 4, insbesondere in dem Fall, wenn A ein 6-gliedriger Aryl- oder Heteroaryl-Ring ist; für den Fall, dass A ein 5-gliedriger Aryl- oder Heteroaryl-Ring ist, ist n bevorzugt 0 bis 4; unabhängig von der Ringgröße ist n besonders bevorzugt 0 bis 3, ganz besonders bevorzugt 0 bis 2 und insbesondere 0 bis 1; dabei tragen die übrigen, keine Substituenten B tragenden Kohlenwasserstoff- oder Heteroatome von A Wasserstoffatome oder gegebenenfalls keinen Substituenten;

B    ist unabhängig voneinander ausgewählt aus der Gruppe bestehend aus Alkyl, Alkenyl, Alkinyl, substituiertem Alkyl, substituiertem Alkenyl, substituiertem Alkinyl, Heteroalkyl, substiuiertem Heteroalkyl, Heteroalkenyl, substituiertem Heteroalkenyl, Heteroalkinyl, substituiertem Heteroalkinyl, Cycloalkyl, Cycloalkenyl, substituiertem Cycloalkyl, substituiertem Cycloalkenyl, Halogen, Hydroxy, Alkoxy, Aryloxy, Carbonyl, Amino, Amido, Thio und Phosphino; bevorzugt ist B unabhängig voneinander ausgewählt aus $C_{1-6}$-Alkyl, $C_{1-6}$-Alkenyl, $C_{1-6}$-Alkinyl, $C_{3-8}$-Cycloalkyl, $C_{3-8}$-Cycloalkenyl, Alkoxy, Aryloxy, Amino und Amido.

Der Ausdruck unabhängig voneinander bedeutet, dass, wenn n 2 oder größer ist, die Substituenten B gleiche oder verschiedene Reste aus den genannten Gruppen sein können.

Unter Alkyl sind gemäß der vorliegenden Anmeldung verzweigte oder unverzweigte, gesättigte acyclische Kohlenwasserstoff-Reste zu verstehen. Bevorzugt werden Alkyl-Reste mit 1 bis 20 Kohlenstoffatomen, besonders bevorzugt mit 1 bis 6 Kohlenstoffatomen und insbesondere mit 1 bis 4 Kohlenstoffatomen eingesetzt. Beispiele für geeignete Alkyl-Reste sind Methyl, Ethyl, n-Propyl, i-Propyl, n-Butyl, t-Butyl und i-Butyl.

Unter Alkenyl sind gemäß der vorliegenden Anmeldung verzweigte oder unverzweigte acyclische Kohlenwasserstoff-Reste zu verstehen, die mindestens eine Kohlenstoff-Kohlenstoff-Doppelbindung aufweisen. Bevorzugt weisen die Alkenyl-Reste 2 bis 20 Kohlenstoffatome, besonders bevorzugt 2 bis 6 Kohlenstoffatome und insbesondere 2 bis 3 Kohlenstoffatome auf. Geeignete Alkenyl-Reste sind beispielsweise Vinyl und 2-Propenyl.

Unter Alkinyl sind gemäß der vorliegenden Anmeldung verzweigte oder unverzweigte acyclische Kohlenwasserstoff-Reste zu verstehen, die mindestens eine Kohlenstoff-Kohlenstoff-Dreifachbindung aufweisen. Vorzugsweise weisen die Alkinyl-Reste 2 bis 20 Kohlenstoffatome, besonders bevorzugt 1 bis 6 Kohlenstoffatome und insbesondere 2 bis 3 Kohlenstoffatome auf. Beispiele für geeignete Alkinyl-Reste sind Ethinyl und 2-Propinyl.

Unter substituiertem Alkyl, substituiertem Alkenyl und substituiertem Alkinyl sind Alkyl-Alkenyl- und Alkinyl-Reste zu verstehen, worin ein oder mehrere Wasserstoffatome, die an ein Kohlenstoffatom dieser Reste gebunden sind, durch eine andere Gruppe ersetzt sind. Beispiele für solche andere Gruppen sind Halogen, Aryl, substituiertes Aryl, Cycloalkyl, Cycloalkenyl, substituiertes Cycloalkyl, substituiertes Cycloalkenyl und Kombinationen davon. Beispiele für geeignete substituierte Alkyl-Reste sind Benzyl und Trifluormethyl.

Unter den Begriffen Heteroalkyl, Heteroalkenyl und Heteroalkinyl sind Alkyl- Alkenyl- und Alkinyl-Reste zu verstehen, worin ein oder mehrere der Kohlenstoffatome in der Kohlenstoffkette durch ein Heteroatom ausgewählt aus N, O und S ersetzt sind. Die Bindung zwischen dem Heteroatom und einem weiteren Kohlenstoffatom kann dabei gesättigt oder ungesättigt sein.

Unter Cycloalkyl sind gemäß der vorliegenden Anmeldung gesättigte cyclische nicht-aromatische Kohlenwasserstoff-Reste zu verstehen, die aus einem einzigen Ring oder mehreren kondensierten Ringen aufgebaut sind. Bevorzugt weisen die Cycloalkyl-Reste zwischen 3 und 8 Kohlenstoffatome und besonders bevorzugt zwischen 3 und 6 Kohlenstoffatome auf. Geeignete Cycloalkyl-Reste sind beispielsweise Cycloproyol, Cyclopentyl, Cyclohexyl, Cyclooctanyl und Bicyclooctyl.

Unter Cycloalkenyl sind gemäß der vorliegenden Anmeldung teilweise ungesättigte, cyclische nicht-aromatische Kohlenwasserstoff-Reste zu verstehen, die einen einzigen oder mehrere kondensierte Ringe aufweisen. Bevorzugt weisen die Cycloalkenyl-Reste 3 bis 8 Kohlenstoffatome und besonders bevorzugt 5 bis 6 Kohlenstoffatome auf. Geeignete Cycloalkenyl-Reste sind beispielsweise Cyclopentenyl, Cyclohexenyl und Cyclooctenyl.

Substituierte Cycloalkyl- und substituierte Cycloalkenyl-Reste sind Cycloalkyl- und Cycloalkenyl-Reste, worin ein oder mehrere Wasserstoffatome eines Kohlenstoffatoms des Kohlenstoffrings durch eine andere Gruppe ersetzt sind. Solche andere Gruppen sind beispielsweise Halogen, Alkyl, Alkenyl, Alkinyl, substituiertes Alkyl, substituiertes Alkenyl, substituiertes Alkinyl, Aryl, substituiertes Aryl, Cycloalkyl, Cycloalkenyl, substituiertes Cycloalkyl, substituiertes Cycloalkenyl, ein aliphatischer heterocyclischer Rest, ein substituierter aliphatischer heterocyclischer Rest, Heteroaryl, substituiertes Heteroaryl, Alkoxy, Aryloxy, Boryl, Phosphino, Amino, Silyl, Thio, Seleno und Kombinationen davon. Beispiele für substituierte Cycloalkyl und Cycloalkenyl-Reste sind 4-Dimethylaminocyclohexyl und 4,5-Dibromocyclohept-4-enyl.

Unter Aryl sind im Sinne der vorliegenden Anmeldung aromatische Reste zu verstehen, die einen einzelnen aromatischen Ring oder mehrere aromatische Ringe aufweisen, die kondensiert sind, über eine kovalente Bindung oder durch eine Verknüpfungseinheit, wie eine Methylen- oder Ethylen-Einheit verknüpft sind. Solche Verknüpfungseinheiten können auch Carbonyl-Einheiten, wie in Benzophenon, oder Sauerstoff-Einheiten, wie in Diphenylether, oder Stickstoff-Einheiten, wie in Diphenylamin, sein. Bevorzugt weisen die Aryl-Reste 6 bis 20 Kohlenstoffatome, besonders bevorzugt 6 bis 8 Kohlenstoffatome und insbesondere bevorzugt 6 Kohlenstoffatome auf. Beispiele für aromatische Ringe sind Phenyl, Naphthyl, Diphenyl, Diphenylether, Diphenylamin und Benzophenon.

Substituierte Aryl-Reste sind Aryl-Reste, worin ein oder mehrere Wasserstoffatome, die an Kohlenstoffatome des Aryl-Rests gebunden sind, durch eine oder mehrere Gruppen wie Alkyl, Alkenyl, Alkinyl, substituiertes Alkyl, substituiertes Alkenyl, substituiertes Alkinyl, Cycloalkyl, Cycloalkenyl, substituiertes Cycloalkyl, substituiertes Cycloalkenyl, Heterocyclo, substituiertes Hetereocyclo, Halogen, halogensubstituiertes Alkyl (z. B. $CF_3$), Hydroxy, Amino, Phosphino, Alkoxy und Thio ersetzt wird. Darüber hinaus können ein oder mehrere Wasserstoffatome, die an Kohlenstoffatome des Aryl-Rests gebunden sind, durch eine oder mehrere Gruppen wie gesättigte und/oder ungesättigte cyclische Kohlenwasserstoffe, die an den aromatischen Ring bzw. an die aromatischen Ringe kondensiert sein können oder durch eine Bindung verknüpft sein können, oder über eine geeignete Gruppe miteinander verknüpft sein können, ersetzt werden. Geeignete Gruppen sind die vorstehend beschriebenen.

Unter Heteroaryl sind gemäß der vorliegenden Anmeldung die vorstehend genannten Arylverbindungen zu verstehen, worin ein oder mehrere Kohlenstoffatome des Rests durch ein Heteroatom, z. B. N, O oder S ersetzt sind.

Unter Heterocyclo ist gemäß der vorliegenden Anmeldung ein gesättigter, teilweise ungesättigter oder ungesättigter cyclischer Rest zu verstehen, worin ein oder mehrere Kohlenstoffatome des Rests durch ein Heteroatom wie N, O oder S ersetzt sind. Beispiele für Heterocyclo-Reste sind Piperazinyl, Morpholinyl, Tetrahydropyranyl, Tetrahydrofuranyl, Piperidinyl, Pyrolidinyl, Oxazolinyl, Pyridyl, Pyrazyl, Pyridazyl, Pyrimidyl.

Substituierte Heterocyclo-Reste sind solche Heterocyclo-Reste, worin ein oder mehrere Wasserstoffatome, die an eines der Ringatome gebunden sind, durch eine oder mehrere Gruppen wie Halogen, Alkyl, substituiertes Alkyl, Aryl, substituiertes Aryl, Heteroaryl, substituiertes Heteroaryl, Alkoxy, Aryloxy, Boryl, Phosphino, Amino, Silyl, Thio, Seleno ersetzt sind.

Unter Alkoxy-Resten sind Reste der allgemeinen Formel $-OZ^1$ zu verstehen, worin $Z^1$ ausgewählt ist aus Alkyl, substituiertem Alkyl, Cycloalkyl, substituiertem Cycloalkyl, Heterocycloalkyl, substituiertem Heterocycloalkyl und Silyl. Geeignete Alkoxy-Reste sind beispielsweise Methoxy, Ethoxy, Benzyloxy und t-Butoxy.

Unter dem Begriff Aryloxy sind solche Reste der allgemeinen Formel $-OZ^2$ zu verstehen, worin $Z^2$ ausgewählt ist aus Aryl, substituiertem Aryl, Heteroaryl, substituiertem Heteroaryl und Kombinationen davon. Geeignete Aryloxy-Reste und Heteroaryloxy-Reste sind Phenoxy, substituiertes Phenoxy, 2-Pyridinoxy und 8-Chinolinoxy.

Unter Amino-Resten sind Reste der allgemeinen Formel $-NZ^3Z^4$ zu verstehen, worin $Z^3$ und $Z^4$ unabhängig voneinander ausgewählt sind aus Wasserstoff, Alkyl, substituiertem Alkyl, Cycloalkyl, substituiertem Cycloalkyl, Heterocycloalkyl, substituiertem Heterocycloalkyl, Aryl, substituiertem Aryl, Heteroaryl, substituiertem Heteroaryl, Alkoxy, Aryloxy und Silyl.

Bevorzugte in dem erfindungsgemäßen Aminierungsverfahren eingesetzte aromatische und heteroaromatische Kohlenwasserstoffe sind Benzol, Naphthalin, Diphenylmethane, Anthracen, Toluol, Xylol, Phenol und Anilin sowie Pyridin, Pyrazin, Pyridazin, Pyrimidin und Chinolin.

In einer bevorzugten Ausführungsform wird demnach mindestens ein Kohlenwasserstoff aus der Gruppe Benzol, Naphthalin, Diphenylmethane, Anthracen, Toluol, Xylol, Phenol und Anilin sowie Pyridin, Pyrazin, Pyridazin, Pyrimidin und Chinolin eingesetzt.

Es ist auch möglich, Mischungen der genannten aromatischen bzw. heteroaromatischen Kohlenwasserstoffe einzusetzen. Besonders bevorzugt wird mindestens ein aromatischer Kohlenwasserstoff aus der Gruppe Benzol, Naphthalin, Anthracen, Toluol, Xylol Phenol und Anilin, ganz besonders bevorzugt Benzol, Toluol, und Naphthalin eingesetzt.

Insbesondere bevorzugt wird Benzol in dem erfindungsgemäßen Verfahren eingesetzt.

Als aliphatischer Kohlenwasserstoff ist Methan für den Einsatz im erfindungsgemäßen Verfahren insbesondere bevorzugt.

Aminierungsreagenz:

Erfindungsgemäß enthält der Eduktstrom E mindestens ein Aminierungsreagenz. Geeignete Aminierungsreagenzien sind solche, durch die mindestens eine Aminogruppe in den zur Direktaminierung eingesetzten Kohlenwasserstoff eingeführt wird. Beispiele für bevorzugte Aminierungsreagenzien sind Ammoniak, primäre und sekundäre Amine und Verbindungen, die unter den Reaktionsbedingungen Ammoniak abspalten. Es ist auch möglich Mischungen von zwei oder mehreren der vorstehend genannten Aminierungsreazien einzusetzen.

Insbesondere bevorzugtes Aminierungsreagenz ist Ammoniak.

Aminokohlenwasserstoffe:

Im erfindungsgemäßen Verfahren wird ein Eduktstrom E, der mindestens einen Kohlenwasserstoff und mindestens ein Aminierungsreagenz enthält, zu einem Reaktionsgemisch R, das mindestens einen Aminokohlenwasserstoff und Wasserstoff enthält, umgesetzt. Dabei wird mindestens ein zum eingesetzten Kohlenwasserstoff korrespondierender Aminokohlenwasserstoff erhalten, der mindestens eine Aminogruppe mehr als der eingesetzte Kohlenwasserstoff enthält. Unter Aminokohlenwasserstoff ist im Rahmen der vorliegenden Erfindung demnach das Reaktionsprodukt der im Verfahren eingesetzten Kohlenwasserstoffe mit dem Aminierungsreagenz zu verstehen. Hierbei wird mindestens eine Aminogruppe von dem Aminierungsreagenz auf den Kohlenwasserstoff übertragen. In einer bevorzugten Ausführungsform werden 1 bis 6 Aminogruppen, in einer besonders bevorzugten Ausführungsform 1 bis 3 Aminogruppen, ganz besonders bevorzugt 1 bis 2 Aminogruppen und insbesondere bevorzugt 1 Aminogruppe auf den Kohlenwasserstoff übertragen. Die Anzahl der übertragenen Aminogruppen lässt sich durch das molare Verhältnis zwischen Aminierungsreagenz und zu aminierendem Kohlenwasserstoff sowie durch die Reaktionstemperatur steuern.

Typischerweise beträgt das Verhältnis von Aminierungsreagenz zu Kohlenwasserstoff 0,5 bis 9, bevorzugt 1 bis 5, besonders bevorzugt 1,5 bis 3.

Für den Fall, dass in das erfindungsgemäße Verfahren als Kohlenwasserstoff Benzol und als Aminierungsreagenz Ammoniak in einem Molverhältnis im Bereich von 1 bis 9 eingesetzt werden, wird als Aminokohlenwasserstoff Anilin erhalten.

Für den Fall, dass in das erfindungsgemäße Verfahren als Kohlenwasserstoff Toluol und als Aminierungsreagenz Ammoniak in einem Molverhältnis im Bereich von 1 bis 9 eingesetzt wird, wird als Aminokohlewasserstoff Toluoldiamin erhalten.

Für den Fall, dass in das erfindungsgemäße Verfahren als Kohlenwasserstoff Methan und als Aminierungsreagenz Ammoniak in einem Molverhältnis im Bereich von 1 bis 9 eingesetzt wird, wird als Aminokohlewasserstoff Methylamin, Dimethylamin oder Trimethylamin oder ein Gemisch aus zwei oder mehreren der vorstehend genannten Amine erhalten.

In einer besonderen Ausführungsform wird Benzol mit Ammoniak zu Anilin umgesetzt. In einer weitern besondern Ausführungsform wird Toluol mit Ammoniak zu Toluoldiamin umgesetzt.


Katalysatoren:

Die Direktaminierung wird in Gegenwart mindestens eines Katalysators durchgeführt.

Als Katalysatoren sind prinzipiell alle bekannten Aminierungskatalysatoren geeignet. Als Katalysatoren können die für die direkte Aminierung von Kohlenwasserstoffen, insbesondere die für die direkte Aminierung von Benzol mit Ammoniak zu Anilin bekannten Katalysatoren eingesetzt werden. Derartige Katalysatoren sind in der Patentliteratur beispielsweise in WO 2007/099028, WO 2007/096297, WO 2000/69804, WO 2008/009668, WO 2007/025882, US 3,919,155, US 3,929,889, US 4,001,260, US 4,031,106, und WO 99/10311 beschrieben. Da gemäß dem erfindungsgemäßen Verfahren die Entfernung des Wasserstoffs elektrochemisch und nicht durch chemische Umwandlung im Reaktionssystem erfolgt, können auch Katalysatoren eingesetzt werden, die keine gegenüber Wasserstoff reaktive Komponenten aufweisen.

Als Katalysatoren können beispielsweise übliche Metallkatalysatoren auf der Basis von Nickel, Eisen, Kobalt, Kupfer, Edelmetallen oder Legierungen dieser genannten Metalle eingesetzt werden. Bevorzugte Edelmetalle (EM) sind Ru, Rh, Pd, Ag, Ir, Pt und Au. In einer besonderen Ausführungsform werden die Edelmetalle Ru und Rh nicht alleine, sondern in Legierung mit einem oder mehreren anderen Übergangsmetallen, wie Co, Cu, Fe und Nickel eingesetzt. Beispiele für geeignete Katalysatoren sind geträgerte NiCuEM; CoCuEM; NiCoCuEM, NiMoEM, NiCrEM, NiReEM, CoMoEM, CoCrEM, Co-ReEM, FeCuEM, FeCoCuEM, FeMoEM, FeReEM Legierungen. Dabei ist EM ein Edelmetall, bevorzugt Pt, Pd, Ag, Ir, insbesondere bevorzugt Ag und/oder Ir. Weiter insbesondere bevorzugt ist NiCuEM, wobei EM aus Pt, Pd, Ag und/oder Ir ausgewählt ist. In einer Ausführungsform dient der auf der Retentatseite eingesetzte Elektrokatalysator (Elektrode) gleichzeitig als Katalysator für die Umsetzung von Kohlenwasserstoff zu Aminokohlenwasserstoff (Aminierungskatalysator). In einer weiteren Ausführungsform kann der Aminierungskatalysator direkt auf dem Elektrokatalysator angebracht werden. In diesem Fall wird der bei der Reaktion freigesetzte Wasserstoff direkt von der Katalysatoroberfläche des Aminierungskatalysators durch die protonenleitende Membran abgeführt.

Der Katalysator kann in allgemein üblicher Form, beispielsweise als Pulver oder für den Einsatz im Festbett in Form von Strängen, Kugeln, Tabletten oder Ringe eingesetzt werden. Die katalytisch aktiven Bestandteile können auf einem Trägermaterial vorliegen. Als Trägermaterial kommen beispielsweise anorganische Oxide, beispielsweise $ZrO_2$, $SiO_2$, $Al_2O_3$, $MgO$, $TiO_2$, $B_2O_3$, $CaO$, $ZnO$, $BaO$, $ThO_2$, $CeO_2$, $Y_2O_3$ und Mischungen dieser Oxide, wie Magnesium-Aluminium-oxid, bevorzugt $TiO_2$ $ZrO_2$, $Al_2O_3$, Magnesium-Aluminium-oxid und $SiO_2$ in Betracht. Besonders bevorzugt sind $Al_2O_3$, $ZrO_2$ und Magnesium-Aluminium-oxid. Unter $ZrO_2$ wird sowohl reines $ZrO_2$ als auch das normale Hf-enthaltende $ZrO_2$ verstanden.

Die in dem erfindungsgemäßen Verfahren bevorzugt eingesetzten Katalysatoren können regeneriert werden, z.B. indem man eine reduktive Atmosphäre über den Katalysator leitet oder zuerst eine oxidative und anschließend eine reduktive Atmosphäre über oder durch das Katalysatorbett führt. Als reduktive Atmosphäre wird vorzugsweise eine $H_2$-Atmosphäre verwendet.

Reaktionsbedingungen Direktaminierung:

Die Direktaminierung kann unter oxidativen oder nicht oxidativen Bedingungen durchgeführt werden. Die Direktaminierung kann darüber hinaus unter katalytischen oder nicht katalytischen Bedingungen durchgeführt werden.

In einer bevorzugten Ausführungsform findet die Direktaminierung in Gegenwart eines Katalysators unter nicht-oxidativen Bedingungen statt.

Nicht-oxidativ gemäß der vorliegenden Erfindung bedeutet in Bezug auf die Direktaminierung, dass die Konzentration von Oxidationsmitteln wie Sauerstoff oder Stickoxiden in den eingesetzten Edukten (Eduktstrom E) unterhalb von 5 Gew.-%, bevorzugt unterhalb von 1 Gew.-%, besonders bevorzugt unterhalb von 0,1 Gew.-% liegt (jeweils bezogen auf das Gesamtgewicht des Eduktstroms E). Unter Eduktstrom E wird im Rahmen der vorliegenden Erfindung der Strom verstanden, der zum Reaktor geführt wird und mindestens einen Kohlenwasserstoff und mindestens ein Aminierungsreagenz enthält. Ganz besonders bevorzugt ist der Eduktstrom E frei von Sauerstoff. Ebenfalls besonders bevorzugt ist eine Konzentration an Oxidationsmittel im Eduktstrom E, die gleichgroß oder geringer ist als die Konzentration an Oxidationsmitteln in der Quelle, aus der die eingesetzten Kohlenwasserstoffe und Aminierungsreagenzien stammen.

Die Reaktionsbedingungen in den erfindungsgemäßen Direktaminierungsverfahren sind unter anderem von dem zu aminierenden Kohlenwasserstoff und dem eingesetzten Katalysator abhängig.

Die Direktaminierung erfolgt im Allgemeinen bei Temperaturen von 20 bis 800 °C, bevorzugt 50 bis 700 °C, besonders bevorzugt 70 bis 350 °C.

Der Reaktionsdruck beträgt bei der Direktaminierung vorzugsweise 0,5 bis 40 bar, bevorzugt 1 bis 6 bar, besonders bevorzugt 1 bis 3 bar, insbesondere Atmosphärendruck.

Die Verweilzeit bei diskontinuierlicher Verfahrensführung beträgt in dem erfindungsgemäßen Aminierungsverfahren im Allgemeinen 15 Minuten bis 8 Stunden, bevorzugt 15 Minuten bis 4 Stunden, besonders bevorzugt 15 Minuten bis 1 Stunde. Bei Durchführung in einem kontinuierlichen Verfahren beträgt die Verweilzeit im Allgemeinen 0,1 Sekunden bis 20 Minuten, bevorzugt 0,5 Sekunden bis 10 Minuten. Für die bevorzugten kontinuierlichen Verfahren bedeutet "Verweilzeit" in diesem Zusammenhang die Verweilzeit des Eduktstroms E am Katalysator, für Festbettkatalysatoren somit die Verweilzeit im Katalysatorbett, für Wirbelschichtreaktoren wird der Syntheseteil des Reaktors (Teil des Reaktors, wo der Katalysator lokalisiert ist) betrachtet.

Die relative Menge des eingesetzten Kohlenwasserstoffs und des Aminierungsreagenz ist abhängig von der durchgeführten Aminierungsreaktion und den Reaktionsbedingungen. Im Allgemeinen werden mindestens stöchiometrische Mengen des Kohlenwasserstoffs und des Aminnierungsreagenz eingesetzt. Bevorzugt wird einer der Reaktionspartner im stöchiometrischen Überschuss einsetzen, um eine Gleichgewichtsverschiebung auf die Seite des gewünschten Produkts und somit einen höheren Umsatz zu erreichen. Bevorzugt wird das Aminnierungsreagenz im stöchiometrischen Überschuss im Bezug auf den Kohlenwasserstoff eingesetzt. Das Verhältnis Aminierungsreagenz zu Kohlenwasserstoff beträgt 0,5 bis 9, bevorzugt 1 bis 5, besonders bevorzugt 1,5 bis 3.

Wasserstoffabtrennung:

Im erfindungsgemäßen Verfahren wird in Schritt b) mindestens ein Teil des im Reaktionsgemisch R enthaltenen Wasserstoffs elektrochemisch abgetrennt. Unter dem Reaktionsgemisch R ist das Gemisch zu verstehen, das durch die chemische Umsetzung von mindestens einem Kohlenwasserstoff mit mindestens einem Aminierungsreagenz entsteht. Das Reaktionsgemisch enthält daher üblicherweise als Reaktionsprodukte den oder die korrespondierenden Aminokohlenwasserstoffe und Wasserstoff. Gegebenenfalls kann das Reaktionsgemisch R darüber hinaus nicht umgesetzte Edukte enthalten. Der Wasserstoff wird mittels einer gasdichten Membran-Elektroden-Assembly abgetrennt, wobei der abzutrennende Wasserstoff in Form von Protonen durch die Membran transportiert wird.

Die Elektroden mit der dazwischen angeordneten Membran werden Membran-Elektroden-Assembly (MEA) genannt. Das Reaktionsgemisch R wird auf einer Seite der Membran entlang geführt. Diese Seite wird im Folgenden Retentatseite genannt. Die andere Seite der Membran wird im Folgenden als Permeatseite bezeichnet. Erfindungsgemäß weist die MEA mindestens eine selektiv protonenleitende Membran auf. Die Membran weist auf jeder Seite mindestens einen Elektrodenkatalysator auf, wobei im Rahmen dieser Beschreibung der auf der Retentatseite befindliche Elektrodenkatalysator als Anodenkatalysator, der auf der Permeatseite befindliche Elektrodenkatalysator als Kathodenkatalysator bezeichnet wird. Auf der Retentatseite wird der Wasserstoff am Anodenkatalysator zu Protonen oxidiert, diese durchqueren die Membran und werden auf der Permeatseite am Kathodenkatalysator gemäß Alternative b1) zu Wasserstoff reduziert und/oder gemäß Alternative b2) zu Wasser umgesetzt.

Für den Transport der Protonen durch die Membran muss gemäß Alternative b1) elektrische Energie aufgewendet werden, die durch Anlegen einer Gleichspannung an die beiden Seiten der Membran mittels Elektroden zugeführt wird.

Die Abtrennung des Wasserstoffs gemäß Alternative b1) wird demnach durch die Ausbildung eines Potentialunterschiedes zwischen den beiden Seiten der Membran durch Anlegen einer Spannung herbeigeführt. Die Abtrennung wird bei Spannungen von 0,05 bis 2000 mV, bevorzugt von 100 bis 900 mV, besonders bevorzugt von 100 bis 800 mV, gemessen gegen eine Wasserstoffreferenzelektrode (RHE) durchgeführt.

Gemäß Alternative b2) wird auf der Permeatseite der Membran ein Sauerstoff enthaltender Strom O entlanggeführt. Gemäß Alternative b2) reagieren auf der Permeatseite die Protonen am Kathaodenkatalysator mit dem Sauerstoff zu Wasser. Die treibende Kraft ist die Reduktion des Sauerstoffs. Bei der Gesamtreaktion wird Energie in Form von Wärme und durch Zwischenschalten eines Verbrauchers in Form von elektrischem Strom frei.

Die Abtrennung des Wasserstoffs nach dem erfindungsgemäßen Verfahren in Schritt b) kann sowohl gemäß Variante b1) als auch gemäß Variante b2) bei Temperaturen von 20 bis 800 °C, bevorzugt von 50 bis 700 °C, besonders bevorzugt von 70 bis 350 °C durchgeführt werden. Bei Verwendung von MEAs basierend auf Polybenzimidazol und Phosphorsäure wird die Abtrennung bevorzugt bei 130 bis 200 °C vorgenommen. Bei der Verwendung von keramischen Membranen, z.B. auf Basis von Ammoniumpolyphosphat, kann bei Temperaturen von 250 bis 350 °C gearbeitet werden.

Die Abtrennung des Wasserstoffs in Schritt b) nach dem erfindungsgemäßen Verfahrens wird vorzugsweise bei Drücken von 0,5 bis 10 bar, bevorzugt von 1 bis 6 bar, besonders bevorzugt von 1 bis 3 bar, insbesondere bei Atmosphärendruck vorgenommen. Gemäß einer bevorzugten Ausführungsform der Erfindung liegt der Druckunterschied zwischen der Retentat- und der Permeatseite der Membran unter 1 bar, bevorzugt unter 0,5 bar, besonders bevorzugt besteht kein Druckunterschied.

Erfindungsgemäß wird mindestens ein Teil des bei der Direktaminierung entstandenen Wasserstoffs abgetrennt. Bevorzugt werden mindestens 30 %, besonders bevorzugt mindestens 50 %, besonders bevorzugt mindestens 70 % und ganz besonders bevorzugt mindestens 95 %, insbesondere mindestens 98 % abgetrennt.

Der auf der Permeatseite gemäß Alternative b1) erhaltene Wasserstoff enthält höchstens 5 Mol-%, bevorzugt höchstens 2 Mol-% und besonders bevorzugt höchstens 1 Mol-% Kohlenwasserstoffe.

Der Wasserstoff kann je nach verwendeter selektiv Protonen leitenden Membran bis zu 30 Mol-%, bevorzugt bis zu 10 Mol-%, besonders bevorzugt bis zu 2 Mol-% Wasser enthalten. Die Gegenwart von Wasser ist bei einigen Membrantypen, zum Beispiel bei bestimmten Polymermembranen zur Befeuchtung der Polymermembran erforderlich.

Die elektrochemische Abtrennung des Wasserstoffs gemäß Schritt b) findet erfindungsgemäß außerhalb der Reaktionszone, in der Schritt a) durchgeführt wird, statt.

Gemäß einer bevorzugten Ausführungsform wird die Abtrennung des Wasserstoffs aus dem Reaktionsgemisch R (Schritt b)) in einem Reaktor durchgeführt, der mit mindestens einer gasdichten MEA in der Art ausgestattet ist. Die Abtrennung kann in einer Ausführungsform beispielsweise in einem Reaktor, dessen Außenwände zumindest teilweise aus gasdichten MEAs gebildet sind, durchgeführt werden.

Der Wasserstoff kann erfindungsgemäß nach Alternative b1), nach Alternative b2) oder nach beiden Alternativen abgetrennt werden. Letzteres bedeutet, dass mindestens ein Teils des Wasserstoffs als Wasserstoff und mindestens ein Teil des Wasserstoffs unter Erzeugung von elektrischer Energie als Wasser erhalten wird. Wie viel des im Reaktionsgemisch R enthaltenen Wasserstoffs jeweils nach Alternative b1) und b2) abgetrennt wird, kann vom Anwender nach Bedarf angepasst werden. Gemäß einer bevorzugten Ausführungsform der Erfindung wird bei der Abtrennung des Wasserstoffs nach Alternative b1) und b2) mindestens soviel Wasserstoff gemäß Alternative b2) abgetrennt, dass der dabei erzeugte Strom ausreicht, um den Energiebedarf für die Wasserstoffabtrennung gemäß Alternative b1) zu decken.

Wird der Wasserstoff aus dem Reaktionsgemisch R gemäß beiden Alternativen b1) und b2) abgetrennt, so wird dies vorzugsweise räumlich getrennt durchgeführt, da bei Gegenwart von Sauerstoff auf der Permeatseite üblicherweise die Protonen direkt zu Wasserstoff reagieren. Das Reaktionsgemisch kann beispielsweise nacheinander zunächst an einer MEA entlang geleitet werden, die auf der Permeatseite mit einem Strom O in Kontakt steht, so dass ein Teil des Wasserstoffs als Wasser abgetrennt wird. Anschließend wird das Reaktionsgemisch R an einer MEA entlang geführt, an die eine Spannung angelegt ist, so dass der Wasserstoff als Wasserstoff abgetrennt wird. Die räumliche Trennung zwischen den beiden Alternativen b1) und b2) kann auch darin bestehen, dass das Reaktionsgemisch R zwischen zwei beispielsweise sich gegenüberliegenden Membranen hindurchgeleitet wird, von denen eine auf der Permeatseite mit einem Strom O in Kontakt steht und an die andere eine Spannung angelegt wird. Dabei befinden sich die MEAs gemäß einer bevorzugten Ausführungsform in dem Reaktor, in dem das Reaktionsgemisch R gebildet wird und besonders bevorzugt bilden die MEAs mindestens einen Teil der räumlichen Begrenzung der Reaktionszone, in der die zum Reaktionsgemisch R führende Reaktion stattfindet.

Der abgetrennte Wasserstoff kann vor der Weiterverwendung noch getrocknet werden, dies wird insbesondere durchgeführt, wenn die Abtrennung durch eine Polymermembran erfolgt, die befeuchtet werden muss.

Reaktoren:

Im erfindungsgemäßen Verfahren können in Schritt a) alle Reaktortypen eingesetzt werden, die für Direktaminierungsreaktionen geeignet sind.

Geeignete Reaktoren sind somit Rohr-Reaktoren, Festbettreaktoren, Wirbelschichtreaktoren, Wanderbetten, zirkulierende Wirbelschichten, Salzbadreaktoren, Plattenwärmetauscher-Reaktoren, Hordenreaktoren mit mehreren Horden mit/oder ohne Wärmetausch bzw. Abzug/Zufuhr von Teilströmen zwischen den Horden, in möglichen Ausführungen als Radialstrom- oder Axialstromreaktoren, wobei jeweils der für die gewünschten Reaktionsbedingungen (wie Temperatur, Druck und Verweilzeit) geeignete Reaktor eingesetzt wird. Die Reaktoren können jeweils als einzelner Reaktor (single reactor), als Serie von einzelnen Reaktoren und/oder in Form von zwei oder mehr parallelen Reaktoren eingesetzt werden. Das erfindungsgemäße Verfahren kann als semi-kontinuierliche Reaktion oder kontinuierliche Reaktion durchgeführt werden. Der spezielle Reaktoraufbau und die Durchführung der Reaktion können in Abhängigkeit von dem durchzuführenden Aminierungsverfahren, dem Aggregatzustand des zu aminierenden aromatischen Kohlenwasserstoffs, den erforderlichen Reaktionszeiten und der Natur des eingesetzten Katalysators variieren. Bevorzugt wird das erfindungsgemäße Verfahren zur Direktaminierung in einem Festbettreaktor oder Wirbelschichtreaktor durchgeführt.

Elektrodenkatalysatoren:

In Schritt b) des erfindungsgemäßen Verfahrens können alle dem Fachmann bekannten Elektrodenkatalysatoren eingesetzt werden.

Die Funktion von Elektrodenkatalysators wird z. B. in Journal of Power Sources 177 (2008), 478-484, K.A. Perry, G.A. Eisman, B.C. Benicewicz "Electrochemical hydrogen pumping using a high-temperature polybenzimidazole (PBI) membrane" beschrieben.

Um einen guten Kontakt der Membran mit dem auf der Retentatseite befindlichen Wasserstoff und einen guten Abtransport des abgetrennten Wasserstoffs auf der Permeatseite zu gewährleisten, sind die Elektrodenschichten üblicherweise mit Gasverteilerschichten kontaktiert. Diese sind zum Beispiel Platten mit einer gitterartigen Oberflächenstruktur aus einem System feiner Kanäle oder Schichten aus porösem Material wie Vlies, Gewebe oder Papier. Die Gesamtheit von Gasverteilerschicht und Elektrodenschicht wird im Allgemeinen als Gasdiffusionselektrode (GDE) bezeichnet. Durch die Gasverteilerschicht wird der abzutrennende Wasserstoff auf der Retentatseite nahe an die Membran und den Anodenkatalysator geführt und auf der Permeatseite der Abtransport des gebildeten Wasserstoffs erleichtert.

Je nach Ausführungsform der Erfindung kann die Anode gleichzeitig auch als Anodenkatalysator und die Kathode gleichzeitig auch als Kathodenkatalysator dienen. Es können jedoch auch jeweils unterschiedliche Materialien für die Anode und den Anodenkatalysator bzw. die Kathode und den Kathodenkatalysator verwendet werden.

In einer Ausführungsform der Erfindung kann der Anodenkatalysator gleichzeitig auch als Aminierungskatalysator dienen. In diesem Fall wird der Anodenkatalysator aus mindestens einem Material der vorstehend genannten Aminierungskatalysatoren ausgebildet.

Zur Herstellung der Elektroden können die üblichen, dem Fachmann bekannten Materialien verwendet werden, beispielsweise Pt, Pd, Cu, Ni, Ru, Co, Cr, Fe, Mn, V, W, Wolframcarbid, Mo, Molybdäncarbid, Zr, Rh, Ag, Ir, Au, Re, Y, Nb, elektrisch leitende Formen von Kohlenstoff wie Ruß, Graphit und Nanoröhrchen sowie Legierungen und Mischungen der vorstehend genannten Elemente.

Die Anode und die Kathode können aus dem gleichen Material oder aus unterschiedlichen Materialien hergestellt sein. Der Anodenkatalysator und der Kathodenkatalysator können aus den gleichen oder aus unterschiedlichen Materialien ausgewählt sein. Besonders bevorzugt sind als Anoden/Kathoden-Kombination Pt/Pt, Pd/Pd, Pt/Pd, Pd/Pt, Pd/Cu, Pd/Ag, Ni/Pt, Ni/Ni und Fe/Fe.

Als Elektrodenkatalysatormaterial können die üblichen, dem Fachmann bekannten Verbindungen und Elemente verwendet werden, die die Dissoziation von molekularem Wasserstoff in atomaren Wasserstoff, die Oxidation von Wasserstoff zu Protonen sowie die Reduktion von Protonen zu Wasserstoff katalysieren können. Geeignet sind beispielsweise Pd, Pt, Cu, Ni, Ru, Fe, Co, Cr, Mn, V, W, Wolframcarbid, Mo, Molybdäncarbid, Zr, Rh, Ag, Ir, Au, Re, Y, Nb sowie Legierungen und Mischungen davon, bevorzugt sind erfindungsgemäß Pd, Pt und Ni. Die vorstehend als Elektrodenkatalysatormaterial genannten Elemente und Verbindungen können auch geträgert vorliegen, bevorzugt wird dabei Kohlenstoff als Träger eingesetzt.

Gemäß einer bevorzugten Ausführungsform der vorliegenden Erfindung werden bevorzugt Kohlenstoff als leitendes Material enthaltende Elektroden verwendet. Dabei wird der Kohlenstoff und ein Elektrodenkatalysator bevorzugt auf einem porösen Träger wie Vlies, Gewebe oder Papier aufgebracht. Der Kohlenstoff kann dabei mit dem Katalysator vermischt oder zunächst der Kohlenstoff und anschließend der Katalysator aufgebracht werden.

Gemäß einer weiteren Ausführungsform der Erfindung wird das als Elektrode eingesetzte elektrisch leitende Material und der Elektrodenkatalysator direkt auf der Membran aufgebracht.

Membranen:

Die Membran in Schritt b) ist vorzugsweise als Platte oder als Rohr ausgebildet, wobei die üblichen, aus dem Stand der Technik zur Auftrennung von Gasgemischen bekannten Membrananordnungen verwendet werden können beispielsweise Rohrbündel- oder Steckplattenmembran.

Die erfindungsgemäß eingesetzte MEA ist gasdicht, das heißt sie weist praktisch keine Porosität auf, durch die Gase in atomarer oder molekularer Form von einer Seite auf die andere Seite der MEA gelangen können, noch weist sie Mechanismen auf, durch die Gase unselektiv beispielsweise durch Adsorption, Lösung in der Membran, Diffusion und Desorption durch die MEA transportiert werden können. Die Dichtigkeit der Membran-Elektroden-Assembly (MEA) kann entweder durch eine gasdichte Membran, durch eine gasdichte Elektrode, oder einen gasdichten Elektrodenkatalysator sowie durch eine Kombination davon gewährleistet werden. So kann als gasdichte Elektrode zum Beispiel eine dünne metallische Folie verwendet werden, beispielsweise eine Pd-, Pd-Ag- oder Pd-Cu-Folie. Weiterhin leitet die erfindungsgemäß eingesetzte Membran selektiv Protonen, das heißt insbesondere, dass sie nicht elektronenleitend ist.

Erfindungsgemäß können für die Membranen alle dem Fachmann bekannten Materialien eingesetzt werden, aus denen sich selektiv protonenleitende Membranen formen lassen. Hierzu gehören beispielsweise die von J. W. Phair und S. P. S. Badwal in Ionics (2006) 12, Seiten 103 - 115 aufgeführten Materialien. Auch selektiv protonenleitende Membranen, wie sie aus der Brennstoffzellentechnik bekannt sind, können erfindungsgemäß verwendet werden.

Beispielsweise können die nachfolgenden keramischen Membranen wie bestimmte Heteropolysäuren wie $H_3Sb_3B_2O_{14} \cdot 10 H_2O$, $H_2Ti_4O_9 \cdot 12 H_2O$ und $HSbP_2O_8 \cdot 10 H_2O$; saure Zirkoniumsilikate, -phosphate und -phosphonate in Schichtstruktur wie $K_2ZrSi_3O_9$, $K_2ZrSi_3O_9$, alpha-$Zr(HPO_4)_2 \cdot nH_2O$, gamma-$Zr(PO_4)$-$(H_2PO_4) \cdot 2H_2O$, alpha- und gamma-Zr-Sulfophenylphosphonat oder Sulfoarylphosphonat; nicht geschichtete Oxidhydrate wie Antimonsäure ($Sb_2O_5 \cdot 2H_2O$), $V_2O_5 \cdot nH_2O$, $ZrO_2 \cdot nH_2O$, $SnO_2 \cdot nH_2O$ und $Ce(HPO_4)_2 \cdot nH_2O$ eingesetzt werden. Weiterhin können Oxosäuren und Salze, die zum Beispiel Sulfat-, Selenat-, Phosphat-, Arsenat-, Nitrat-Gruppen usw. enthalten, verwendet werden. Besonders geeignet sind Oxoanionensysteme auf Basis von Phosphaten oder komplexen Heteropolysäuren wie Polyphosphatgläser, Aluminiumpolyphosphat, Ammoniumpolyphosphat und Polyphosphatzusammensetzungen wie $NH_4PO_3/(NH_4)_2SiP_4O_{13}$ und $NH_4PO_3/TiP_2O_7$. Weiterhin sind oxidische Materialien einsetzbar, wie Brownmillerite, Fluorite und Phosphate mit Apatitstruktur, Pyrochlor-Mineralien und Perovskite. Generell können alle protonenleitenden Materialien, wie z.B. auch Zeolithe, Alumosilikate, $xAl_2O_3(1-x)SiO_2$, $SnP_2O_7$, $Sn_{1-x}In_xP_2O_7$ (X = 0.0 - 0.2), eingesetzt werden.

Perovskite weisen die Grundformel $AB_{1-x}M_xO_{3-y}$ auf, wobei M ein trivalentes Seltenerdenelement ist, das zur Dotierung dient, und y den Sauerstoffmangel im Perovskitoxid-Gitter bezeichnet. A kann beispielsweise ausgewählt werden aus Mg, Ca, Sr und Ba. B kann unter anderem ausgewählt werden aus CeZr und Ti. Für A, B und M können auch unabhängig voneinander verschiedene Elemente aus den jeweiligen Gruppen gewählt werden.

Weiterhin können strukturell modifizierte Gläser eingesetzt werden wie Chalcogenid-Gläser, $PbO$-$SiO_2$, $BaO$-$SiO_2$ und $CaO$-$SiO_2$.

Weitere geeignete protonenleitende Keramiken und Oxide sind beispielsweise in Solid State Ionics 125, (1999), 271-278; Journal of Power Sources 180, (2008), 15-22; Ionics 12, (2006), 103-115; Journal of Power Sources 179 (2008) 92-95; Journal of Power Sources 176 (2008) 122-127 und Electrochemistry Communications 10 (2008) 1005-1007 beschrieben.

Beispiele für protonenleitende Keramiken und Oxide sind $SrCeO_3$, $BaCeO_3$, $Yb:SrCeO_3$, $Nd:BaCeO_3$, $Gd:BaCeO_3$, $Sm:BaCeO_3$, $BaCaNd0_9$, $Y:BaCeO_3$, $Y:BaZrCeO_3$, Pr-dotiertes $Y:BaCeO_3$, $Gd:BaCeO_3$, $BaCe_{0,9}Y_{0,1}O_{2,95}$ (BYC), $SrCe_{0,95}Yb_{0,05}O_{3-\alpha}$, $BaCe_{0,9}Nd_{0,10}O_{3-\alpha}$, $CaZr_{0,96}In_{0,04}O_{3-\alpha}$, ($\alpha$ bezeichnet die Anzahl der Sauerstofffehlstellen pro Formeleinheit des Oxides vom Perowskittyp); Sr-dotiertes $La_3P_3O_9$, Sr-dotiertes $LaPO_4$, $BaCe_{0,9}Y_{0,1}O_{3-\alpha}$ (BCY), $BaZr_{0,9}Y_{0,1}O_{3-\alpha}$ (BZY), $Ba_3Ca_{1,18}Nb_{1,82}O_{8,73}$ (BCN18), $(La_{1,95}Ca_{0,05})Zr_2O_{7-\alpha}$, $La_2Ce_2O_7$, $Eu_2Zr_2O_7$, $H_2S/(B_2S_3$ oder $Ga_2S_3)/GeS_2$, $SiS_2$, $As_2S_3$ oder CsI; $BaCe_{0,8}Gd_{0,2}O_{3-\alpha}$ (BCGO); Gd-dotiertes $BaCeO_3$ wie $BaCe_{0,85}Y_{0,15}O_{3-\alpha}$ (BCY15) und $BaCe_{0,8}Sm_{0,2}O_{3-\alpha}$, $xAl_2O_3(1-x)SiO_2$, $SnP_2O_7$, $Sn_{1-x}In_xP_2O_7$ (x = 0.0 - 0.2).

Eine weitere Klasse Materialien, die sich für die Herstellung von gasdichten und selektiv protonenleitenden Membranen eignen, sind Polymermembranen. Geeignete Polymere sind sulfonierte Polyetheretherketone (S-PEEK), sulfonierte Polybenzoimidazole (S-PBI) und sulfonierte Fluorkohlenwasserstoffpolymere (NAFION®). Weiterhin können perflurierte Polysulfonsäuren, Polymere auf Styrolbasis, Poly(arylenether), Polyimide und Polyphosphazene eingesetzt werden. Es können auch Polybenzamidazolen eingesetzt werden, die auf Polybenzimidazol und Phosphorsäure basieren, wie sie beispielsweise unter dem Namen Celtec-P® von der BASF SE vertrieben werden.

Erfindungsgemäß werden als Materialien für die selektiv protonenleitende Membran bevorzugt die vorstehend genannten keramischen Membranen eingesetzt.

Bei Verwendung von Polymermembranen werden diese üblicherweise durch die Gegenwart von etwa 0,5 bis 30 Vol.-% Wasser auf mindestens einer Seite der Membran befeuchtet.

Aufarbeitung des Produktstroms:

Die Abtrennung des Wasserstoffs aus dem Reaktionsgemisch R gemäß Schritt b) erfolgt mittels mindestens einer MEA. Nach Abtrennung des Wasserstoffs aus dem Reaktionsgemisch R enthält dieses in einer bevorzugten Ausführungsform weniger als 500, bevorzugt weniger als 200 und insbesondere bevorzugt weniger als 100 ppm Wasserstoff.

Nach der Abtrennung des Wasserstoffs aus dem Reaktionsgemisch R mittels mindestens einer MEA wird ein Produktstrom P erhalten. Dieser enthält mindestens einen Aminokohlenwasserstoff und gegebenenfalls nicht umgesetzte Edukte, wie Kohlenwasserstoffe und Aminierungsreagenzien, sowie gegebenenfalls nicht abgetrennten Wasserstoff. In einer bevorzugten Ausführungsform enthält der Produktstrom P weniger als 500, bevorzugt weniger als 200 und insbesondere bevorzugt weniger als 100 ppm Wasserstoff.

Die Abtrennung von Aminokohlenwasserstoff und/oder Aminierungsreagenz kann nach allgemein bekannten, dem Fachmann geläufigen Methoden erfolgen, beispielsweise durch Kondensation, Destillation oder Extraktion. Die Wahl des Druck- und Temperaturbereichs richtet sich nach den physikalischen Eigenschaften der aufzutrennenden Verbindungen und ist dem Fachmann bekannt.

Gemäß einer Ausführungsform werden die entstandenen Aminokohlenwasserstoffe zwischen Schritt a) und b) aus dem Reaktionsgemisch abgetrennt. Gemäß einer weiteren Ausführungsform der Erfindung werden die entstandenen aromatischen Kohlenwasserstoffe nach Schritt b) aus dem Reaktionsgemisch R abgetrennt. Gemäß einer weiteren Ausführungsform der Erfindung werden die entstandenen aromatischen Kohlenwasserstoffe nach Schritt b) aus dem Produktstrom P abgetrennt.

In einer besonders bevorzugten Ausführungsform der Erfindung wird das Reaktionsgemisch R nach Abtrennung der Aminokohlenwasserstoffe und/oder des Aminierungsreagenz und mindestens eines Teils des Wasserstoffs in das Verfahren zurückgeführt, das Reaktionsgemisch R wird entweder dem Eduktstrom E oder direkt in die Reaktionszone der Direktaminierung zurückgeführt.

Die Erfindung wird durch die nachstehenden Beispiele näher erläutert, ohne sie darauf zu beschränken.

Beispiel 1:

In einem Aminierungsreaktor mit 1 cm Innendurchmesser und 50 cm Länge werden 20 ml Ni-Cu/Al$_2$O$_3$ Katalysator als Festbett angeordnet. Anschließend werden bei einer Temperatur von 350°C und einem Druck von 40 bar 68 g/h Benzol und 44 g/h Ammoniak (Eduktstrom E) in den Aminierungsreaktor eingetragen. Als Reaktoraustrag (Reaktionsgemisch R) erhält man ein Gemisch aus Benzol, Anilin (im Flüssigaustrag wurden 99,5 % Benzol und 0,42 % Anilin gefunden), 3900 ppm Wasserstoff, 1100 ppm Stickstoff und Ammoniak (Rest).

Der Reaktoraustrag wird in einen nach gelagerten Reaktor (Elektrozelle) mit einer gasdichten MEA mit 45 cm$^2$ aktiver Fläche geleitet. Als Membran der MEA wird CeltecP$^®$ (Polyimidazol/Phosphorsäure) der BASF Fuel Cell GmbH verwendet. Als Elektroden der MEA werden sowohl für Anode als auch für Kathode Palladium Folien mit einer Dicke von 10 µm eingesetzt

Die Elektrozelle wird bei 190°C betrieben. An der MEA wird eine Spannung ($U_A$) von +250 mV angelegt. Der Wasserstoff wird auf der Retentatseite an der Anode zu Protonen oxidiert, diese durchqueren die Membran und werden an der Kathode zu Wasserstoff reduziert.

Aus dem Reaktoraustrag des Aminierungsreaktors wird in der Elektrozelle ein Großteil des enthaltenden Wasserstoffs abgetrennt. Der Reaktoraustrag aus der Elektrozelle wird zurück in den Aminierungsreaktor geleitet.

Beispiel 2

In einer Elektrozelle bestehend aus einer gasdichten MEA mit 45 cm$^2$ aktiver Fläche wurden 80 l/h Stickstoff und 0,5 l/h Wasserstoff (Wasserstoffkonzentration 6200 ppm) eingetragen. Als Membran der MEA wird CeltecP$^®$ (Polyimidazol/Phosphorsäure) der BASF Fuel Cell GmbH verwendet. Als Elektroden der MEA werden sowohl für Anode als auch für Kathode Gasdiffussionselektroden ELAT (1 mg/cm$^2$ Platin) der BASF Fuel Cell GmbH eingesetzt. Die Elektrozelle wurde bei 190°C und 1 bar Druck betrieben. An der MEA lag eine Spannung ($U_A$) von +250 mV an.

Im Austrag der Elektrozelle waren 40 ppm Wasserstoff und Stickstoff (Rest) vorhanden. Dies entspricht einer Wasserstoffabtrennung von 99,4 %.

Beispiel 3

In Tabelle 1 wird die Effektivität der Wasserstoffabtrennung in Beispiel 2 mit Vergleichsbeispielen aus dem Stand der Technik verglichen.

Tabelle 1

| Beispiel | Material MEA | Temperatur | Spannung | Wasserstoffkonzentration | | Druckunterschied Retentat/Permeatseite | | H$_2$ Permeance |
|---|---|---|---|---|---|---|---|---|
| | | °C | mV | Eintrag | Austrag | bar | Pa | mol/m$^2$.s . Pa |
| Beispiel 2 | Pt-GDE/Celtec P | 190 | 250 | 6200 ppm | 40 ppm | 0,00062 | 62 | 1,99E-05 |
| Vergleichsbeispiel 2 | Sol-Gel Si/AlO$_x$ | 200 | | | | | | 5,00E-07 |
| Vergleichsbeispiel 3 | Pd-Ag/a-Al$_2$O$_3$ | 200-340 | | | | 0,8-2,5 | 100000 | 1,40E-06 |
| Vergleichsbeispiel 4 | Pd | 200 | | | | 0,51 | 51000 | 5,20E-07 |

*Vergleichsbeispiel 2 Reference # 55 in* Ind. Eng. Chem. Res. 2006, 45, 875-881;
*Vergleichsbeispiel 3 Reference # 189 in* Top. Catal. (2008), 51: 107-122;
*Vergleichsbeispiel 4 Reference # 158 in* Top. Catal. (2008), 51: 107-122.

Tabelle 1 zeigt, dass der mit dem erfindungsgemäßen Verfahren erzielbare Wasserstofffluss ($H_2$-Permeance mol/$m^2$ x s x Pa) durch die Membran im Vergleich zu den herkömmlichen im Stand der Technik beschriebenen Verfahren deutlich erhöht ist.

**Patentansprüche**

1.  Verfahren zur Direktaminierung von Kohlenwasserstoffen zu Aminokohlenwasserstoffen umfassend die Schritte:

    a) Umsetzen eines Eduktstroms E, der mindestens einen Kohlenwasserstoff und mindestens ein Aminierungsreagenz enthält, zu einem Reaktionsgemisch R enthaltend Aminokohlenwasserstoffe und Wasserstoff, und
    b) elektrochemische Abtrennung mindestens eines Teils des bei der Umsetzung entstandenen Wasserstoffs aus dem Reaktionsgemisch R mittels einer gasdichten Membran-Elektroden-Assembly, die mindestens eine selektiv protonleitende Membran und auf jeder Seite der Membran mindestens einen Elektrodenkatalysator aufweist, wobei auf der Retentatseite der Membran mindestens ein Teil des Wasserstoffs an dem Anodenkatalysator zu Protonen oxidiert wird und die Protonen nach Durchqueren der Membran auf der Permeatseite an dem Kathodenkatalysator gemäß

    b1) zu Wasserstoff reduziert werden und/oder
    b2) mit Sauerstoff zu Wasser umgesetzt werden, wobei der Sauerstoff aus einem Sauerstoff enthaltenden Strom O stammt, der mit der Permeatseite der Membran in Kontakt gebracht wird.

2.  Verfahren gemäß Anspruch 1, **dadurch gekennzeichnet, dass** die entstandenen Aminokohlenwasserstoffe zwischen Schritt a) und Schritt b) aus dem Reaktionsgemisch R abgetrennt werden.

3.  Verfahren gemäß Anspruch 1, **dadurch gekennzeichnet, dass** die entstandenen Aminokohlenwasserstoffe nach Schritt b) aus dem Reaktionsgemisch R abgetrennt werden.

4.  Verfahren gemäß einem der Ansprüche 1 bis 3, **dadurch gekennzeichnet, dass** das Reaktionsgemisch R nach Abtrennung mindestens eines Teils des entstandenen Wasserstoffs und der Aminokohlenwasserstoffe in das Verfahren zurückgeführt wird.

5.  Verfahren gemäß einem der Ansprüche 1 bis 4, **dadurch gekennzeichnet, dass** aus dem Reaktionsgemisch R mindestens 30 %, bevorzugt mindestens 50 %, besonders bevorzugt mindestens 70 %, ganz besonders bevorzugt mindestens 95 % und insbesondere bevorzugt mindestens 98 % des im Reaktionsgemisch R enthaltenen Wasserstoffs abgetrennt werden.

6.  Verfahren gemäß einem der Ansprüche 1 bis 5, **dadurch gekennzeichnet, dass** die Wasserstoffabtrennung bei Temperaturen von 20 bis 800 °C, bevorzugt bei 50 bis 700, und insbesondere bevorzugt von 70 bis 350 °C durchgeführt wird.

7.  Verfahren gemäß einem der Ansprüche 1 bis 6, **dadurch gekennzeichnet, dass** die Wasserstoffabtrennung gemäß Alternative b1) bei Spannungen von 0,05 bis 2000 mV, bevorzugt von 100 bis 900 mV, besonders bevorzugt von 100 bis 800 mV, durchgeführt wird.

8.  Verfahren gemäß einem der Ansprüche 1 bis 7, **dadurch gekennzeichnet, dass** der Sauerstoff enthaltende Strom O gemäß Alternative b2) mindestens 15 Mol-% Sauerstoff enthält.

9.  Verfahren gemäß einem der Ansprüche 1 bis 8, **dadurch gekennzeichnet, dass** bei gleichzeitiger Abtrennung des Wasserstoffs gemäß b1) und b2) mindestens ein Teil des bei b2) erzeugten Stroms in b1) eingesetzt wird.

10.  Verfahren gemäß einem der Ansprüche 1 bis 9, **dadurch gekennzeichnet, dass** die Elektroden der Membran-Elektroden-Assembly als metallische Folien ausgestaltet sind.

11.  Verfahren gemäß einem der Ansprüche 1 bis 10, **dadurch gekennzeichnet, dass** bei Abtrennung des Wasserstoffs gemäß b2) mindestens ein Teil der bei b2 entstehenden Wärme der Reaktionszone zugeführt wird.

12.  Verfahren gemäß einem der Ansprüche 1 bis 11, **dadurch gekennzeichnet, dass** die Elektroden der Membran-Elektroden-Assembly als Gasdiffusionselektroden ausgestaltet sind.

13. Verfahren gemäß einem der Ansprüche 1 bis 11, **dadurch gekennzeichnet, dass** die Elektroden der Membran-Elektroden-Assembly als metallische Folien ausgestaltet sind.

14. Verfahren gemäß einem der Ansprüche 1 bis 13, **dadurch gekennzeichnet, dass** als selektiv protonenleitende Membran Membranen ausgewählt aus der Gruppe Keramikmembranen und Polymembranen eingesetzt werden.

15. Verfahren gemäß einem der Ansprüche 1 bis 14, **dadurch gekennzeichnet, dass** als Kohlenwasserstoff Benzol, Toluol oder Methan eingesetzt wird und als Aminokohlenwasserstoff Anilin, Toluyldiamin oder Methylamin entsteht.

**Claims**

1. A process for the direct amination of hydrocarbons to aminohydrocarbons, which comprises the steps:

   a) reaction of a feed stream E comprising at least one hydrocarbon and at least one aminating reagent to form a reaction mixture R comprising aminohydrocarbons and hydrogen and
   b) electrochemical separation of at least part of the hydrogen formed in the reaction from the reaction mixture R by means of a gastight membrane-electrode assembly having at least one selectively proton-conducting membrane and at least one electrode catalyst on each side of the membrane, where at least part of the hydrogen is oxidized to protons over the anode catalyst on the retentate side of the membrane and the protons are, after passing through the membrane,

   b1) reduced to hydrogen and/or
   b2) reacted with oxygen from an oxygen-comprising stream 0 which is brought into contact with the permeate side of the membrane to form water

   over the cathode catalyst on the permeate side.

2. The process according to claim 1, wherein the aminohydrocarbons formed are separated off from the reaction mixture R between step a) and step b).

3. The process according to claim 1, wherein the aminohydrocarbons formed are separated off from the reaction mixture R after step b).

4. The process according to any of claims 1 to 3, wherein the reaction mixture R after at least part of the hydrogen formed and the aminohydrocarbons have been separated off is recirculated to the process.

5. The process according to any of claims 1 to 4, wherein at least 30%, preferably at least 50%, particularly preferably at least 70%, very particularly preferably at least 95% and in particular at least 98%, of the hydrogen comprised in the reaction mixture R is separated off from the reaction mixture R.

6. The process according to any of claims 1 to 5, wherein the hydrogen removal is carried out at temperatures of from 20 to 800°C, preferably from 50 to 700°C and particularly preferably from 70 to 350°C.

7. The process according to any of claims 1 to 6, wherein the hydrogen removal according to alternative b1) is carried out at potentials of from 0.05 to 2000 mV, preferably from 100 to 900 mV, particularly preferably from 100 to 800 mV.

8. The process according to any of claims 1 to 7, wherein the oxygen-comprising stream 0 in alternative b2) comprises at least 15 mol% of oxygen.

9. The process according to any of claims 1 to 8, wherein, when the hydrogen is simultaneously separated off according to b1) and b2), at least part of the stream produced in b2) is used in b1).

10. The process according to any of claims 1 to 9, wherein the electrodes of the membrane-electrode assembly are configured as metallic foils.

**11.** The process according to any of claims 1 to 10, wherein, when the hydrogen is separated off according to b2), at least part of the heat produced in b2) is fed to the reaction zone.

**12.** The process according to any of claims 1 to 11, wherein the electrodes of the membrane-electrode assembly are configured as gas diffusion electrodes.

**13.** The process according to any of claims 1 to 11, wherein the electrodes of the membrane-electrode assembly are configured as metallic foils.

**14.** The process according to any of claims 1 to 13, wherein membranes selected from the group consisting of ceramic membranes and polymer membranes are used as selectively proton-conducting membrane.

**15.** The process according to any of claims 1 to 14, wherein benzene, toluene or methane is used as hydrocarbon and aniline, toluenediamine or methylamine is formed as aminohydrocarbon.


**Revendications**

**1.** Procédé pour l'amination directe d'hydrocarbures en aminohydrocarbures comprenant les étapes de :

a) transformation d'un flux de produits de départ E, qui contient au moins un hydrocarbure et au moins un réactif d'amination, en un mélange réactionnel R contenant des aminohydrocarbures et de l'hydrogène et
b) séparation électrochimique d'au moins une partie de l'hydrogène formé lors de la transformation du mélange réactionnel R au moyen d'un ensemble d'électrode à membrane imperméable aux gaz, qui présente au moins une membrane guidant les protons de manière sélective et au moins un catalyseur d'électrode sur chaque côté de la membrane, au moins une partie de l'hydrogène étant oxydée en protons côté retentat de la membrane sur le catalyseur anodique et les protons, après avoir traversé la membrane étant, sur le côté perméat du catalyseur cathodique,

b1) réduits en hydrogène et/ou
b2) transformés avec de l'oxygène en eau, l'oxygène provenant d'un flux 0 contenant de l'oxygène qui est mis en contact avec le côté perméat de la membrane.

**2.** Procédé selon la revendication 1, **caractérisé en ce que** les aminohydrocarbures formés sont séparés du mélange réactionnel R entre l'étape a) et l'étape b).

**3.** Procédé selon la revendication 1, **caractérisé en ce que** les aminohydrocarbures formés sont séparés du mélange réactionnel R après l'étape b).

**4.** Procédé selon l'une quelconque des revendications 1 à 3, **caractérisé en ce que** le mélange réactionnel R est recyclé dans le procédé après séparation d'au moins une partie de l'hydrogène formé et des aminohydrocarbures formés.

**5.** Procédé selon l'une quelconque des revendications 1 à 4, **caractérisé en ce qu'**on sépare du mélange réactionnel R au moins 30%, de préférence au moins 50%, de manière particulièrement préférée au moins 70%, de manière tout particulièrement préférée au moins 95% et en particulier de préférence au moins 98% de l'hydrogène contenu dans le mélange réactionnel R.

**6.** Procédé selon l'une quelconque des revendications 1 à 5, **caractérisé en ce que** la séparation de l'hydrogène est réalisée à des températures de 20 à 800°C, de préférence de 50 à 700°C, en particulier de préférence de 70 à 350°C.

**7.** Procédé selon l'une quelconque des revendications 1 à 6, **caractérisé en ce que** la séparation de l'hydrogène selon l'alternative b1) est réalisée à des tensions de 0,05 à 2000 mV, de préférence de 100 à 900 mV, de manière particulièrement préférée de 100 à 800 mV.

**8.** Procédé selon l'une quelconque des revendications 1 à 7, **caractérisé en ce que** le flux 0 contenant de l'oxygène selon l'alternative b2) contient au moins 15% en mole d'oxygène.

**9.** Procédé selon l'une quelconque des revendications 1 à 8, **caractérisé en ce que**, lors de la séparation simultanée de l'hydrogène selon b1) et b2), au moins une partie du flux obtenu lors de b2) est utilisée dans bl).

**10.** Procédé selon l'une quelconque des revendications 1 à 9, **caractérisé en ce que** les électrodes de l'ensemble d'électrode à membrane sont conçues comme des feuilles métalliques.

**11.** Procédé selon l'une quelconque des revendications 1 à 10, **caractérisé en ce que**, lors de la séparation de l'hydrogène selon b2), au moins une partie de la chaleur formée lors de b2) est introduite dans la zone de réaction.

**12.** Procédé selon l'une quelconque des revendications 1 à 11, **caractérisé en ce que** les électrodes de l'ensemble d'électrode à membrane sont conçues comme électrodes à diffusion de gaz.

**13.** Procédé selon l'une quelconque des revendications 1 à 11, **caractérisé en ce que** les électrodes de l'ensemble d'électrode à membrane sont conçues comme des feuilles métalliques.

**14.** Procédé selon l'une quelconque des revendications 1 à 13, **caractérisé en ce qu'**on utilise, comme membrane guidant les protons de manière sélective, des membranes choisies dans le groupe formé par les membranes céramiques et les membranes polymères.

**15.** Procédé selon l'une quelconque des revendications 1 à 14, **caractérisé en ce qu'**on utilise, comme hydrocarbure, du benzène, du toluène ou du méthane et, comme aminohydrocarbure, de l'aniline, de la toluyldiamine ou de la méthylamine.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007096297 A
- WO 200069804 A
- WO 2007099028 A
- WO 2008009668 A
- WO 2007025882 A
- US 3919155 A
- US 3929889 A
- US 4001260 A
- US 4031106 A
- WO 9910311 A

**In der Beschreibung aufgeführte Nicht-Patentliteratur**

- *Berichte,* 1917, vol. 50, 541-546
- **BASILE.** *Top. Catal.,* 2008, vol. 51, 107-122
- **K.A. PERRY ; G.A. EISMAN ; B.C. BENICEWICZ.** Electrochemical hydrogen pumping using a high-temperature polybenzimidazole (PBI) membrane. *Journal of Power Sources,* 2008, vol. 177, 478-484
- **J. W. PHAIR ; S. P. S. BADWAL.** *Ionics,* 2006, vol. 12, 103-115
- *Solid State Ionics,* 1999, vol. 125, 271-278
- *Journal of Power Sources,* 2008, vol. 180, 15-22
- **LONICS.** *Ionics,* 2006, vol. 12, 103-115
- *Journal of Power Sources,* 2008, vol. 179, 92-95
- *Journal of Power Sources,* 2008, vol. 176, 122-127
- *Electrochemistry Communications,* 2008, vol. 10, 1005-1007
- *Ind. Eng. Chem. Res.,* 2006, vol. 45, 875-881
- *Top. Catal.,* 2008, vol. 51, 107-122